# EUROPEAN PATENT SPECIFICATION

(11) **EP 0 315 732 B1**
(45) Date of publication and mention of the grant of the patent: **10.02.1993**
(21) Application number: 88109780.2
(22) Date of filing: 20.06.1988
(51) Int. Cl.: A61F 13/02

(54) **Process for producing first-aid adhesive bandages**
Verfahren zur Herstellung von Heftpflastern für Erste Hilfe
Procédé de fabrication de pansements adhésifs pour premiers soins

(30) Priority: 11.11.1987 JP 172193/87
(43) Date of publication of application: 17.05.1989
(73) Proprietor: ASO PHARMACEUTICAL CO., LTD., Kikuchi-gun Kumamoto-ken (JP)
(72) Inventor: Murata, Takaaki, Kikuyo-cho Kikuchi-gun Kumamoto-ken (JP)
(74) Representative: Barz, Peter, Dr.

(56) References cited:
- GB-A- 350 384
- GB-A- 1 381 855
- US-A- 3 157 178
- US-A- 3 277 891
- US-A- 3 853 598
- US-A- 4 334 530

## Description

This invention relates to a process for producing a first-aid adhesive bandage having a pad applicable to the skin and affixed to the surface of an adhesive coating.

This type of first-aid adhesive bandages finds frequent use in treating a slight wound in fingers, hands, legs, face or other body parts at offices, plants, hospitals, schools, households or elsewhere and is among medical supplies indispensable in daily life.

The first-aid adhesive bandage comprises a flexible backing sheet, an adhesive coating formed on one surface thereof and a pad adhered to the adhesive coating at its center. In use, the bandage is adhered, at the pad, to the skin of a human body with the adhesive and thus may be peeled off. No problem would arise if peeling occurs at, e.g., ordinary offices or plants. But if peeling takes place in, e.g., food-manufacturing plants or sites for manufacture of medicaments, sanitary products, medical supplies or the like, the peeled bandage may get mixed with or attached to the starting materials, or semifinished or finished articles in the production line.

To prevent shipment of articles contaminated with a first-aid adhesive bandage or other undesirable foreign matter, the starting materials and semifinished or finished articles have been subjected to operators' visual inspection for detection thereof at the manufacturing site. Nevertheless, this detecting method has the problems of failing to achieve accurate detection and to notice defiled materials and articles.

To cope with the problem, a first-aid adhesive bandage has been proposed which includes a backing material containing metallic powder dispersed therein or in a coating. (GB-A-1381855). The proposed method contemplates detection of contaminated food or other articles by passage thereof beside a metal detector without resort to visual inspection. With this method, however, the presence of insufficient amount of metallic powder dispersed in the backing material makes it difficult to perform an accurate detection by a metal detector. Particularly for detection of the adhesive bandage existing in food or the like, a substantial amount of metallic powder is required. Further, the dispersed metallic powder is present as a foreign matter in the soft resin used as the component of backing material so that an excessive amount of dispersed metallic powder reduces the strength of backing material. With these problems, it has been difficult to produce first-aid adhesive bandages which are satisfactory both in assuring accurate detection by a metal detector and in retaining the strength of backing material.

It is an object of the present invention to provide a first-aid adhesive bandage free from the abovenoted problems.

More particularly, the object of the invention is to provide a first-aid adhesive bandage which permits accurate detection of materials or articles contaminated with the bandage without visual inspection and which allows desired detection without involving the problem of having the strength of backing material reduced due to the presence of metallic powder.

To achieve the object, the present invention provides a process for producing a first-aid adhesive bandage comprising a sheet of backing material and a coat of adhesive agent formed on the upper surface of the sheet of backing material or on the surface of an undercoat of primer formed on the sheet of backing material, the process being characterized in that metallic powder as a portion to be detected by a metal detector, is deposited by printing with a metallic powder-containing ink on the underside surface of the sheet of backing material.

The first-aid adhesive bandage of the present invention is provided with a portion for detection which a metal detector can detect. For detection, a metal detector check is carried out by causing the starting materials and semifinished or finished articles to pass beside a metal detector arranged in a production line in a plant for manufacture of foods, sanitary goods, medical supplies or the like, or alternatively by bringing the metal detector into close proximity to such materials or articles, whereby the bandage slipped off during operation can be discovered without aid of visual inspection.

According to the present invention, the detection portion is provided as metallic portion deposited on the adhesive-uncoated surface of the backing material. In consequence, even a large amount of metallic powder can be present without affecting the strength of backing material or with the strength thereof retained. In other words, accurate detection by a metal detector is assured by use of metallic powder in a sufficient amount. Further the first-aid adhesive bandage of this invention can retain the desired flexibility of backing material without the likelihood of producing an incongruous feeling in application to the skin and can be used as conveniently as common first-aid adhesive bandages.

Advantageously the first-aid adhesive bandage of the invention may contain metallic powder provided in the form of, for example, a corporate device, a mark, letters or colored design for identification of the manufacturer or distributor so that a first-aid adhesive bandage accidentally detected in a commercial article can be immediately distiguished, thereby providing a visible proof showing, to great advantage, that the manufacturer's or distributor's bandage is not the source of the contamination.

These and other objects and features of the invention will be further clarified by the following description of specific embodiments with reference to the accompanying drawings.

In the drawings:

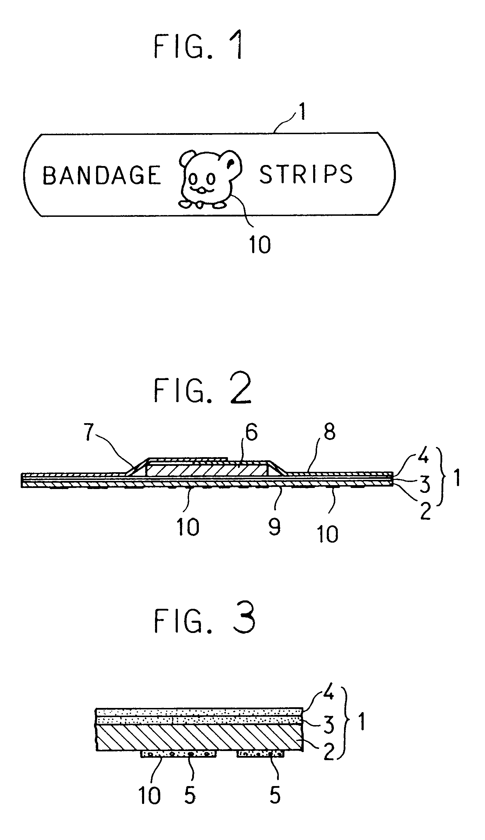

Fig. 1 is a perspective view showing an embodiment of the first-aid adhesive bandage of the invention with the peel paper sheets partly peeled;
Fig. 2 is a side view thereof in vertical section;
Fig. 3 is a partly enlarged side view thereof in vertical section ;

Referring to the drawing, an adhesive sheet 1 comprises an adhesive 4 deposited on an undercoat of a primer 3 on the upper surface of a backing material or a support 2 in the form of soft resin film or fabric, nonwoven fabric or the like cut to a rectangular or like suitable shape.

The primer 3 is applied to strengthen the anchorage of adhesive 4 to the backing material 2.

Indicated at 6 is a pad made of gauze or like material and affixed to the upper surface of adhesive sheet 1 centrally thereof. A pair of peel paper sheets 7, 8 are temporarily adhered at the external ends thereof to the adhesive 4 and have the internal ends thereof overlapped over each other to cover the pad 6.

A metallic portion 10 is provided on an underside 9 of the adhesive sheet 1 without distribution thereof in the primer 3 nor in the adhesive 4. The underside 9 on the adhesive sheet 1 is not coated with the adhesive 4 and thus is exposed in use.

According to this embodiment, a suitable device, mark, letters or colored design is printed with a magnetic ink to form the metallic portion 10. Usable as a magnetic material 5 to be incorporated in the magnetic ink are e.g. powders of γ-type ferric oxide or tri-iron tetroxide which have the property of being readily perceived by a metal detector. The magnetic material can be mixed with an ink base or pigment to give a magentic ink, which is used to form the metallic portion 10 as stated above by proper printing means such as offset printing or letterpress printing.

Alternatively the metallic portion 10 can be produced by printing with a metallic powder (e.g. iron powder)-containing ink in place of the magnetic ink or by vapor deposition of aluminum or the like on the underside 9.

The present invention includes first-aid adhesive bandages produced by suitable combinations of procedures described hereinbefore, i.e., inclusion of metallic powder 5 in the primer 3 and/or adhesive 4, printing with the magnetic ink or metallic powder-containing ink on the underside of the adhesive sheet 1, vapor deposition of metal on the underside thereof, etc.

## Claims

1. A process for producing a first-aid adhesive bandage comprising a sheet of backing material (2) and a coat of adhesive agent (4) formed on the upper surface of the sheet of backing material (2) or on the surface of an undercoat of primer (3) formed on the sheet of backing material (2), characterized in that metallic powder (5), as a portion to be detected by a metal detector, is deposited by printing with a metallic powder-containing ink on the underside surface of the sheet of backing material (2).

2. A process according to claim 1, wherein the metallic portion is formed both by printing with a metallic powder-containing ink and by vapor deposition of metal on a surface of the backing material (2) uncoated with adhesive (4).

## Patentansprüche

1. Verfahren zur Herstellung von Erste-Hilfe-Heftpflastern, umfassend eine Trägerschicht (2) und eine Klebstoffschicht (4) auf der oberen Oberfläche der Trägerschicht (2) oder auf der Oberfläche einer Primer-Grundierung, die auf der Trägerschicht (2) ausgebildet ist, dadurch gekennzeichnet, daß Metallpulver (5) als durch einen Metalldetektor nachweisbarer Anteil durch Bedrucken mit einer Metallpulver enthaltenden Farbe auf die untere Oberfläche der Trägerschicht (2) aufgebracht wird.

2. Verfahren nach Anspruch 1, worin der Metallanteil sowohl durch Bedrucken mit einer Metallpulver enthaltenden Farbe als auch durch Aufdampfen von Metall auf die Oberfläche des Trägermaterials (2), die nicht mit dem Klebstoff (4) beschichtet ist, gebildet wird.

## Revendications

1. Procédé de fabrication d'un pansement adhésif de premiers soins comportant une feuille de matériau de support (2) et une couche d'agent adhésif (4) formée sur la surface supérieure de la feuille de matériau de support (2) ou sur la surface d'une couche inférieure de fond (3) formée sur la feuille de matériau de support (2), caractérisé en ce qu'une poudre métallique (5), en tant que partie à détecter par un détecteur de métal, est déposée par impression avec une encre contenant une poudre métallique sur la surface inférieure de la feuille de matériau de support (2).

2. Procédé selon la revendication 1, dans lequel la partie métallique est formée par impression avec une encre contenant une poudre métallique et par dépôt de métal en phase vapeur sur une surface du matériau de support (2) non recouvert de l'adhésif (4).
